(19) Europäisches Patentamt
European Patent Office
Office européen des brevets

(11) Publication number: **0 071 458**
**B1**

(12) **EUROPEAN PATENT SPECIFICATION**

(45) Date of publication of patent specification: **10.10.84**

(51) Int. Cl.$^3$: **C 07 C 29/32**, C 07 C 29/38, C 07 C 31/20

(21) Application number: **82303960.7**

(22) Date of filing: **27.07.82**

(54) **Process for producing ethylene glycol.**

(30) Priority: **28.07.81 US 286721**
**26.02.82 US 352920**

(43) Date of publication of application:
**09.02.83 Bulletin 83/06**

(45) Publication of the grant of the patent:
**10.10.84 Bulletin 84/41**

(84) Designated Contracting States:
**BE CH DE FR GB IT LI NL SE**

(56) References cited:
**GB-A-2 083 037**
**GB-A-2 083 038**
**US-A-4 076 758**

**ANGEWANDTE CHEMIE, vol. 72, no. 21, 7th November 1960, pages 779-80, K. SCHWETLICK et al.: "Reaktion von Di-tert.-butylperoxyd mit prim. und sek. Alkoholen"**

**JOURNAL OF ORGANIC CHEMISTRY, vol. 30, no. 7, July 1965, pages 2429-2432, Easton (USA); M.OYAMA: "A free-radical reaction of primary and secondary alcohols with formaldehyde"**

(73) Proprietor: **Redox Technologies Inc.**
**6 Spencer Court**
**Wyckoff New Jersey (US)**

(72) Inventor: **Kollar, John**
**6 Spencer Court**
**Wyckoff New Jersey (US)**

(74) Representative: **De Minvielle-Devaux, Ian Benedict Peter et al**
**CARPMAELS & RANSFORD 43, Bloomsbury Square**
**London WC1A 2RA (GB)**

**EP 0 071 458 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a process for producing ethylene glycol from methanol.

Dwindling petroleum reserves and increasing prices have placed an increased emphasis on the use of synthesis gas in place of oil as a starting material for producing various chemicals, such as methanol, formaldehyde and ethylene glycol. The advantage of synthesis gas is that it can be produced from raw materials other than petroleum, such as natural gas or coal, and potentially from oil shale and tar sands.

An example of an industrial process for the production of ethylene glycol utilising synthesis gas as a starting material is the reaction of formaldehyde with carbon monoxide and water at high pressures (over 300 atmospheres) in the presence of an acid catalyst to produce hydroxyacetic (glycolic) acid, which is then reacted with methanol to give the methyl ester; the latter is then converted to the glycol by catalytic hydrogenation. See U.S. Patents 2,316,564 of Cockerill; 2,153,064 of Larson and 2,152,852; 2,385,448 and 2,331,094 of Loder.

Another proposed process utilising synthesis gas for the production of ethylene glycol is the reaction of methanol and carbon monoxide using a rhodium-catalysed, high pressure process; see U.S. Patents 4,115,428 of Vidal et al, and 4,115,433 of Cosby et al.

With respect to the type of process for the production of ethylene glycol disclosed and claimed herein, it should be noted that the oxidative dimerisation or dehydrodimerisation of a large variety of organic compounds by peroxides is very old art that was pioneered by the pre-eminent free radical theoretician M. S. Kharasch and his students. These studies became the foundation of much subsequent free radical chemistry. Kharasch et al in *JACS* 65, 15 (1943) show the dehydrodimerisation of acetic acid to succinic acid with acetyl peroxide in a 50 mole percent utilisation selectivity based on acetyl peroxide, utilisation selectivity being defined as the moles of dehydrodimer product made divided by the moles of peroxide converted. Isobutyric acid produced tetramethylsuccinic acid in a 42.4 mole percent utilisation selectivity. Kharash et al in *J. Org. Chem.* 10, 386 (1945) show the ester methyl chloroacetate being dimerised to dimethyldichloro succinate by acetyl peroxide in a 41 percent utilisation selectivity. Kharash et al in *J. Org. Chem.* 10, 401 (1945) show the dimerisation of cumene and ethylbenzene with acetyl peroxide in 61.9 mole percent and 32.1 mole percent respectively to their dehydrodimers. Wiles et al in *I, E & C,* August 1949, page 1682, tell of the efficacy of di-t-butyl peroxide and 2,2bis(t-butylperoxy) butene for the dimerisation of cumene to 1,1,2,2-tetramethyl 1,2-diphenylethane. The benzoate ester of benzoyl alcohol was dimerised to the dibenzoate ester of the corresponding glycol, 1,2-diphenylene ethylene glycol, with di-t-butyl peroxide by Rust et al, *JACS* 70, 3258 (1948).

The literature contains many other examples showing production of dehydrodimers at very low concentrations at utilisation selectivities of generally from 20—50 mole percent, based on the peroxide consumed. Such selectivities are generally too low for a process to be considered for commercial development.

In connection with ethylene glycol, two teachings involving peroxide-induced reactions should be mentioned.

The first is found in Schwetlick et al, *Angew. Chem.* 72, No 21, 779—780 (1960), and involves heating a mixture of di-tertiary-butyl peroxide and methanol in a molar ratio of 1:20 in an autoclave and/or under reflux for a period of 10 hours at 140°C. A 26 percent yield of ethylene glycol is reported, and it is stated that an increase in the alcohol excess raises the yields.

The second and more important of such other reaction paths to ethylene glycol, in terms of its relevance to the present invention, is described bu Oyama in *J. Org. Chem.* 30, 2429—2432 (July 1965). In particular, Oyama shows the reaction of 9 moles of methanol, 1.8 moles of 15 percent aqueous formaldehyde and 0.45 moles of t-butyl peroxide (di-tertiary-butyl peroxide) at 140°C. for 12 hours to give 0.21 moles of ethylene glycol (see Table I at the top of the right-hand column on page 2430) and states immediately below Table I: "The yield of ethylene glycol in the reaction of formaldehyde with methanol is higher than that of t-butyl peroxide induced dimerization of methanol. This fact suggests that hydroxymethyl radical (D) adds to formaldehyde". Oyama described in greater detail how this reaction was run and the products obtained, and contrasts it with the dehydrodimerisation of methanol in the presence of t-butyl peroxide and the absence of formaldehyde, in the "Experimental" section beginning at page 2431 (particularly the sections headed "Reaction of Methanol with Formaldehyde" and Dimerization of Methanol" on page 2432).

The yields of ethylene glycol obtained by Oyama are fairly low. Oyama's only run with methanol (that involving the above-described reaction of methanol, aqueous formaldehyde and t-butyl peroxide at 140°C. for 12 hours) gave only 1.86 weight percent of ethylene glycol.

The above-described reaction can be made to produce higher yields of ethylene glycol by substantially decreasing the amount of organic peroxide employed, relative to the amounts of formaldehyde and methanol present, from that employed by Oyama. Moreover, increasing the amount of methanol and decreasing the amount of water, relative to the other components of the reaction mixture, in contrast to the amounts employed by Oyama, also appear to contribute to the production of higher yields of ethylene glycol. Thus, for example, heating a mixture of 78.5 weight percent of

methanol, 1.5 weight percent of di-tertiary-butyl peroxide, 6.9 weight percent of formaldehyde and 13.1 weight percent of water at 155°C. for 2 hours gave a yield of 4.5 weight percent of ethylene glycol in the product mixture. This is equivalent to a yield of about 7.1 moles of ethylene glycol per mole of di-tertiary-butyl peroxide employed. (Oyama obtained 0.466 mole of ethylene glycol per mole of di-tertiary-butyl peroxide in his reaction). This improvement in which the amount of peroxide employed is up to 6% by weight of the reaction mixture, is new. It is described in a pending Patent application, U.S. Serial No. 183,537, filed September 2, 1980.

It is possible to produce ethylene glycol from methanol and an organic peroxide, alone or in the presence of formaldehyde and water reacted in the presence of a basic material in an amount sufficient to reduce the hydrogen ions that are being formed in the reaction without unduly reducing ethylene glycol production due to by-product formation. The basic material in the reaction reduces the acids, such as formic acid, formed in the reaction which catalyse the formation of methylal from methanol and formaldehyde. Keeping the formation of methylal to minimum is highly desirable in order to avoid unduly large or expensive distillation requirements necessary for the purification of the ethylene glycol product. This procedure is new. It is described in a pending U.S. Patent application, U.S. Serial No. 286,721, filed July 28, 1981.

In accordance with the present invention, ethylene glycol is produced by reacting methanol, an organic peroxide (as defined below) in an amount greater than 6 up to 25 weight percent, formaldehyde, and water in an amount from 0.5 to 35 weight percent, these amounts being based on the total weight of these four reactants in the reaction mixture; the amount of water used is dependent on the amount of peroxide used in accordance with Table A below. The amount of ethylene glycol produced by this process is larger than that produced by the process described in the Oyama reference discussed earlier which employs 40.5% methanol, 7.55% formaldehyde, 9.18% di-tertiary-butyl peroxide and 42.77% water based on the weight of the reaction mixture. It has been discovered in this invention that if the water content of the reaction is reduced in accordance with Table A, substantial improvements in amounts of ethylene glycol to di-tertiary-butyl peroxide (mole ratio basis) are achieved over that of the Oyama reference using peroxide amounts greater than 6 up to 25 percent. In addition to the ethylene glycol improvement, higher amounts of tertiary-butyl alcohol to di-tertiarybutyl peroxide (mole ratio basis) are achieved in the process of this invention compared to the Oyama reference. The mole ratio of acetone to di-tertiary-butyl peroxide was generally higher in the Oyama reference than the acetone/ditertiary-butyl peroxide ratio of the present invention. Tertiary-butyl alcohol production is of particular significance in its use as an intermediate for gasoline additives as well as a gasoline additive itself.

The Drawing is a graph illustrating the correlation of di-tertiary-butyl peroxide (weight percent) with the mole ratio of the ethylene glycol produced to said peroxide. It is further discussed in connection with the Examples.

For purposes of this invention, the amounts (weight percentages) of organic peroxide and water used must be within the range of Table A which follows.

TABLE A

| Peroxide | Water |
|---|---|
| greater than 6 up to 12 | 0.5 to 35 |
| greater than 12 up to 15 | 0.5 to 25 |
| greater than 15 up to 20 | 0.5 to 15 |
| greater than 20 up to 25 | 0.5 to 10 |

The amount of formaldehyde contained in the reaction mixture is normally from 0.5 to 13 weight percent, preferably from 2 to 12 weight percent. The amount of methanol present in the reaction mixture is the amount necessary to make the mixture up to 100%. For example, the ranges of amounts (weight percentages) of reactants in Table B can be used.

TABLE B

| Peroxide | Water | Formaldehyde | Methanol |
|---|---|---|---|
| greater than 6 up to 12 | 0.5—35 | 2—12 | 41—91.5 |
| greater than 12 up to 15 | 0.5—25 | 2—12 | 48—85.5 |
| greater than 15 up to 20 | 0.5—15 | 2—12 | 53—82.5 |
| greater than 20 up to 25 | 0.5—10 | 2—12 | 53—77.5 |

A preferred set of ranges of amounts (weight percentages) is given in Table C.

TABLE C

| Peroxide | Water | Formaldehyde | Methanol |
|---|---|---|---|
| greater than 6 up to 10 | 5—10 | 2—12 | 68—87 |

The reaction will generally be carried out at a temperature of from 100°C to 200°C., preferably from 125°C to 175°C., with a residence time which is not greater than 8 hours, usually from 0.25 to 8 hours, and preferably from 0.5 to 4 hours. Generally, the higher the temperature, the lower the reaction time necessary to bring the reaction to a desired state of completion. There is little or no criticality in the pressure at which the reaction is carried out. In general, pressures from autogenous pressure up to 600 psig ($4{,}137.10^6$ Pa) can be utilised.

The organic peroxide employed in the process of this invention is defined as one which has the formula

$$R—O—O—R^1$$

wherein R and $R^1$ (which may the same or different) are each an alkyl or aralkyl group having 3 to 12 carbon atoms. Organic peroxides which may be employed include, for example, di-tertiary-butyl peroxide, di-cumyl peroxide, tertiary-butyl cumyl peroxide and tertiary-butylethylbenzyl peroxide. The preferred organic peroxide is di-tertiary-butyl peroxide.

An inert solvent may be employed in the process, although in most cases it is preferred to operate without one. Any solvent which does not react under conditions of the process may be employed, e.g. benzene and tertiary-butyl alcohol. When a solvent is employed, it will generally be in an amount up to 25 percent by weight of the total reaction medium, although larger amounts can be used some instances.

The reactions may be carried out batchwise wherein a reactor such as a stirred autoclave is charged with the initial reaction mixture which is then subjected to reaction, after which the entire reaction mixture is withdrawn and purified. Another batchwise processing technique is to charge the methanol, water and portions of the formaldehyde (usually mixed with some water) and peroxide as the initial reaction mixture and then add the remaining portions of the formaldehyde and peroxide and in some cases basic material, which are sequentially and incrementally added to the methanol reaction medium. After all of the portions of reactants are added, the reaction is continued until the desired degree of reaction has taken place, after which the product mixture is withdrawn and purified. Another technique is a semi-continuous method in which the initial reaction mixture and incremental additions of reactants are charged to the methanol reaction medium, reaction takes place and the product mixture periodically withdrawn from the reactor and purified. In this connection, reference is made to our co-pending European Patent Application filed together with the present application and identified as c-5957A/5957B (=EP—A—0071457).

A continuous reaction can be carried out in a reactor constructed so that under the conditions of reaction, a glycol concentration gradient occurs between the point where the first portions of peroxide and formaldehyde are added and that at which the glycol-containing product is withdrawn. Thus subsequent portions of peroxide and formaldehyde are added between these two points. For example, a

continuous process may be carried out by causing the liquid reaction medium to flow through a pipe and adding portions of the formaldehyde and peroxide reactants at intervals along the pipe, forming a moving reaction medium containing a concentration gradient of glycol as it travels through the pipe. Thus, the individual portions of the reactants are added in a controlled manner along the pipe to the specific moving reaction medium until all the portions of the reactants have been added. At the end of the pipe reactor, the reaction of each portion of the reactants in the reaction medium has occurred at a conversion level to provide a product stream containing the desired amount of ethylene glycol. Alternatively a baffled reactor may be employed containing a liquid reaction medium with a glycol concentration gradient between the point of entry of the initial feed stream and the point of exit of the product stream, wherein additional portions of peroxide and formaldehyde are added between the two points. In each case, the product mixture may then be purified using conventional techniques such as distillation or solvent extraction to obtain ethylene glycol in the desired purity, preferably fibre grade, and by-products such as tertiary-butyl alcohol, methylal, methyl formate, glycerine and acetone.

The following Examples illustrate the invention.

Examples 1—14

An initial reaction mixture containing methanol (MeOH), di-tertiary-butyl peroxide (DtBP), formaldehyde ($CH_2O$) and water were charged into a stirred autoclave in the following manner: all but about 20 weight percent of the methanol in the initial charge, formaldehyde and water were added to a stirred autoclave at room temperature. The mixture was then heated up to reaction temperature and a solution of ditertiary-butyl peroxide in the remaining 20 weight percent of methanol was added to the autoclave to initiate the reaction. A reaction pressure of 500 psig ($3,447.10^6$ Pa) was maintained with nitrogen. After a reaction time of one hour, the product mixture was removed from the autoclave and analysed for ethylene glycol (EG), acetone (A), tertiary-butyl alcohol (TBA), unreacted di-tertiary-butyl peroxide and other products present in minor amounts.

The amounts of the reactants and of the products, and the temperature, pressure and reaction time, of each Example are shown in Table I.

Examples 15—18

These were comparative Examples.

As a basis for comparison of this invention with the Oyama reference described previously, Example 15 illustrates the results of the Oyama process. This run was made by changing methanol, di-tertiary-butyl peroxide, formaldehyde and water to 304 stainless steel Hoke reactor at atmospheric pressure. The reactor was capped and placed in a thermostated oil bath held at the stated reaction temperature and allowed to react for one hour at autogenous pressure. After the reaction time was completed, the reactor was cooled by quenching, vented, discharged and analysed for ethylene glycol, acetone, tertiary-butyl alcohol, unreacted di-tertiary-butyl peroxide and other products present in minor amounts.

Examples 16, 17 and 18 illustrate the use of water from 35.7 to 45 weight percent with peroxide, formaldehyde and methanol reacted in a 304 stainless steel Hoke reactor at atmospheric pressure. It is to be noted that in each of these runs 0.015 weight percent sodium bicarbonate was added to the initial reaction mixture. The reactor was capped and placed in a thermostated oil bath held at 155°C. for one hour at autogenous pressure. After the reaction was completed, the reactor was cooled by quenching, vented, discharged and analysed for ethylene glycol, acetone, tertiary-butyl alcohol, unreacted di-tertiary-butyl peroxide and other products present in minor amounts. In Example 16, the analysis of ethylene glycol and unreacted di-tertiary-butyl peroxide was determined but acetone and tertiary-butyl alcohol were not determined.

The results of Examples 14—18 are also shown in Table I, which sets out the composition of the initial charge, temperature, pressure and reaction time employed for the reaction.

The results of Example 15 according to the Oyama reference for ethylene glycol production is similar to the results given in the reference itself. Comparing the results of Example 15 with Examples 1—14 which illustrate the results of the present invention, it should be noted that Examples 1—14 all produce higher mole ratios of ethylene glycol/di-tertiary-butyl peroxide and tertiary-butyl alcohol/di-tertiary butyl peroxide than does the Oyama reference run of Example 15. In regard to acetone, Example 15 produces a higher mole ratio of acetone/di-tertiary-butyl peroxide than the runs of Examples 1—14.

Examples 16—18 illustrate the use of over 35 weight percent of water in the reaction mixture which is outside the scope of this invention. Even though a base, i.e. 0.015 weight percent of sodium bicarbonate, was employed in the reaction mixture which, as mentioned above, has the effect of reducing the formation of by-product methylal and raising the production of ethylene glycol, the amount of glycol produced in Examples 17 and 18 was below that obtained by the Oyama method, but the mole ratio of glycol to di-tertiary-butyl peroxide was higher than in the Oyama method.

In Table II, the yields of the products of all the Examples are expressed as mole ratios based on the di-tertiary-butyl peroxide consumed.

TABLE I

Ethylene Glycol Production

Initial Charge Wt%

| Example | DtBP | $CH_2O$ | MeOH | $H_2O$ | Temperature °C |
|---|---|---|---|---|---|
| 1 | 6 | 10 | 79 | 5 | 155 |
| 2 | 9 | 10 | 46 | 35 | 155 |
| 3 | 12 | 10 | 53 | 25 | 155 |
| 4 | 6 | 10 | 59 | 25 | 155 |
| 5 | 10 | 10 | 65 | 15 | 155 |
| 6 | 15 | 10 | 60 | 15 | 155 |
| 7 | 20 | 10 | 55 | 15 | 155 |
| 8 | 20 | 10 | 60 | 10 | 155 |
| 9 | 15 | 10 | 65 | 10 | 155 |
| 10 | 10 | 10 | 70 | 10 | 155 |
| 11 | 12 | 10 | 43 | 35 | 155 |
| 12 | 25 | 10 | 55 | 10 | 155 |
| 13 | 25 | 10 | 62.7 | 2.3 | 155 |
| 14 | 25 | 10 | 60 | 5 | 155 |
| 15 | 9.3 | 7.6 | 41.1 | 42.0 | 155 |
| 16 | 6.1 | 10.2 | 46 | 35.7 | 155 |
| 17 | 1.5 | 10.0 | 43.5 | 45 | 155 |
| 18 | 6.0 | 6.0 | 43 | 45 | 155 |

TABLE I (cont.)

Ethylene Glycol Production

Products Wt %

| Example | EG | A | TBA | DtBP |
|---|---|---|---|---|
| 1 | 7.90 | 2.24 | 2.70 | 0.25 |
| 2 | 4.10 | 4.72 | 1.92 | 0.29 |
| 3 | 5.35 | 6.50 | 3.20 | 0.58 |
| 4 | 5.20 | 3.38 | 1.94 | 0.26 |
| 5 | 6.70 | 4.90 | 3.90 | 0.48 |
| 6 | 6.70 | 7.00 | 5.60 | 0.71 |
| 7 | 6.20 | 9.99 | 7.60 | 0.90 |
| 8 | 7.00 | 8.80 | 7.90 | 0.98 |
| 9 | 7.00 | 7.35 | 6.60 | 0.59 |
| 10 | 7.38 | 4.20 | 4.19 | 0.58 |
| 11 | 3.93 | 6.43 | 2.41 | 0.67 |
| 12 | 6.05 | 10.8 | 10.5 | 1.50 |
| 13 | 7.36 | 9.73 | 11.7 | 2.10 |
| 14 | 6.89 | 10.5 | 11.1 | 2.70 |
| 15 | 1.59 | 5.50 | 1.73 | 0.03 |
| 16 | 3.20 | — | — | 0.13 |
| 17 | 1.31 | 1.04 | 0.38 | 0.03 |
| 18 | 1.51 | 3.20 | 1.61 | 0.02 |

TABLE II

Ethylene Glycol Production

Product Mole Ratios

| Example | EG/DtBP | A/DtBP | TBA/DtBP |
|---|---|---|---|
| 1 | 3.24 | 1.02 | 0.93 |
| 2 | 1.11 | 1.41 | 0.43 |
| 3 | 1.10 | 1.48 | 0.55 |
| 4 | 2.13 | 1.54 | 0.67 |
| 5 | 1.66 | 1.34 | 0.81 |
| 6 | 1.10 | 1.28 | 0.77 |
| 7 | 0.76 | 1.36 | 0.79 |
| 8 | 0.88 | 1.21 | 0.82 |
| 9 | 1.14 | 1.33 | 0.90 |
| 10 | 1.84 | 1.16 | 0.88 |
| 11 | 0.82 | 1.48 | 0.42 |
| 12 | 0.61 | 1.20 | 0.88 |
| 13 | 0.76 | 1.11 | 1.01 |
| 14 | 0.73 | 1.23 | 0.98 |
| 15 | 0.40 | 1.55 | 0.37 |
| 16 | 1.26 | — | — |
| 17 | 2.10 | 1.84 | 0.51 |
| 18 | 0.59 | 1.40 | 0.53 |

The results of Examples 1—18, illustrating a correlation of di-tertiary-butyl peroxide charged (weight percent) to the mole ratio of ethylene glycol/di-tertiarybutyl peroxide obtained at various levels of water, are plotted on the graph shown in the Drawing. (Example 13 is the only example not plotted on the graph since only one point of a 2.3 weight percent water run is available). The graph illustrates that as the amount of water is decreased, higher amounts of di-tertiary-butyl peroxide can be used to achieve better results than those of the Oyama reference. The graph illustrates the effectiveness of the relationship required by the present invention between the ranges of proportion of peroxide and the ranges of water content.

Examples 19—22

These Examples illustrate the use of equivalent total amounts of reactants used in a single stage reaction and multiple stage reactions to produce ethylene glycol (EG) by the reaction of formaldehyde ($Ch_2O$) containing water ($H_2O$), di-tertiary-butyl peroxide (DtBP) and methanol (MeOH) in the presence of sodium bicarbonate ($NaHCO_3$). The formaldehyde used in these Examples contained 4.54 parts per million sodium hydroxide per one weight percent formaldehyde used. The reactor was a 316 stainless steel 1" (2.54 cm) diameter pipe having a capacity of approximately 85 millimetres liquid. In a one stage reaction, all the reacting ingredients were placed in the reactor which was sealed and heated to 155°C. for one hour at autogenous pressure. In a staged reaction, a portion of the reactants of di-tertiary-butyl peroxide, formaldehyde and water and sodium bicarbonate were initially added to the

reactor containing all the methanol heated to 155°C. for one hour. After the first hour of reaction, additional portions of the reactants were added and the reaction was conducted for another hour. After the second hour, the remainder of the reactants were added to the reactor and the reaction conducted for the final hour. After the reaction was completed in the first stage or in each multiple stage reaction, the reactor was cooled by quenching, vented and discharged, and the contents were analysed by gas chromatography for ethylene glycol (EG) and other products.

The results of these Examples are shown in Table III which sets out the composition of total amounts of peroxide, formaldehyde and water charged to the reactor containing the methanol in the various stages. The amounts of reactants used are reported as weight percent of the total material in the reactor up to that stage. The amounts of ethylene glycol are reported as weight percent of the total product mixture.

TABLE III

| Examples | Type of reaction | | Reactants Added | | | | | |
|---|---|---|---|---|---|---|---|---|
| | | | DtBP Wt% | $H_2CO$ Wt% | $H_2O$ Wt% | $NaHCO_3$ Parts per million | Reaction time hours | Ethylene Glycol Wt% |
| 19 | 1 stage | | 7.0 | 14.0 | 1.91 | 100 | 1 | 8.75 |
| 20 | 3 stage | | | | | | | |
| | | 1st. stage | 3.0 | 6.0 | 0.8 | 50 | 1 | 6.14 |
| | | 2nd stage (total) | 5.0 | 10.0 | 1.36 | 75 | 1+1=2 | 9.28 |
| | | 3rd stage (total) | 7.0 | 14.0 | 1.91 | 100 | 1+1=3 | 11.68 |
| 21 | 1 stage | | 6.42 | 11.77 | 1.60 | 100 | 1 | 8.92 |
| 22 | 3 stage | | | | | | | |
| | | 1st. stage | 3.0 | 7.0 | 0.95 | 50 | 1 | 7.32 |
| | | 2nd. stage (total) | 4.77 | 9.48 | 1.33 | 75 | 1+1=2 | 9.58 |
| | | 3rd stage (total) | 6.42 | 11.77 | 1.60 | 100 | 2 + 1=3 | 11.48 |

**Claims**

1. A process for producing ethylene glycol by reacting methanol, an organic peroxide and formaldehyde in the presence of water, said organic peroxide having the formula R—O—O—R[1] wherein each of R and R[1] (which may be the same or different) is an alkyl or aralkyl group containing 3 to 12 carbon atoms, characterised in that the amount of the peroxide used is greater than 6 and up to 25 weight percent and the amount of water used is from 0.5 to 35 weight percent, and the amounts of the peroxide and water used are dependent on each other so that either the amount of the peroxide used is greater than 6 up to 12 weight percent and the amount of water used is from 0.5 to 35 weight percent, or the amount of the peroxide used is greater than 12 up to 15 weight percent and the amount of water used is from 0.5 to 25 weight percent, or the amount of the peroxide used is greater than 15 up to 20 weight percent and the amount of water used is from 0.5 to 15 weight percent, or the amount of the peroxide used is greater than 20 up to 25 weight percent and the amount of water used is from 0.5 to 10 weight percent, the above weight percentages being based on the total weight of the methanol, organic peroxide, formaldehyde and water present in the reaction mixture.

2. The process of claim 1 wherein the organic peroxide is di-tertiary-butyl peroxide.

3. The process of claim 1 or 2 wherein the reaction time is no greater than 8 hours.

4. The process of claim 3 wherein the initial reaction mixture contains di-tertiary-butyl peroxide as the peroxide, water, 0.5 to 13 weight percent formaldehyde and the remainder is methanol, the reaction temperature is from 100 to 200°C. and the reaction time is from 0.25 to 8 hours.

5. The process of claim 4 wherein the amount of formaldehyde is 2 to 12 weight percent, the reaction temperature is from 125°C. to 175°C and the reaction time is from 0.5 to 4 hours.

6. The process of claim 5 wherein the initial reaction mixture contains from 41 to 91.5 weight percent of methanol, greater than 6 up to 12 weight percent of di-tertiary-butyl peroxide and from 0.5 to 35 weight percent water.

7. The process of claim 5 wherein the initial reaction mixture contains from 48 to 85.5 weight percent of methanol, greater than 12 up to 15 weight percent of di-tertiary-butyl peroxide and from 0.5 to 25 weight percent of water.

8. The process of claim 5 wherein the initial reaction mixture contains from 53 to 82.5 weight percent of methanol, greater than 15 up to 20 weight percent of di-tertiary-butyl peroxide and from 0.5 to 15 weight percent water.

9. The process of claim 5 wherein the initial reaction mixture contains from 53 to 77.5 weight percent of methanol, greater than 20 up to 25 weight percent of di-tertiary-butyl peroxide and from 0.5 to 10 weight percent water.

10. The process of claim 5 wherein the initial reaction mixture contains from 68 to 87 weight percent of methanol, greater than 6 up to 10 weight percent of di-tertiary-butyl peroxide and from 5 to 10 weight percent water.

**Revendications**

1. Procédé de synthèse de l'éthylène glycol par réaction de méthanol, d'un peroxyde organique et de formaldéhyde en présence d'eau, ledit peroxyde organique ayant pour formule R—O—O—R[1] où chacun de R et R[1] (qui peuvent être identiques ou différents) est un groupe alcoyle ou aralcoyle contenant 3 à 12 atomes de carbone, caractérisé en ce que la quantité du peroxyde utilisée est supérieure à 6 et peut atteindre 25% en poids et la quantité d'eau utilisée est comprise entre 0,5 et 35% en poids et les quantités du peroxyde et de l'eau utilisées dépendent l'une de l'autre donc, soit la quantité de peroxyde utilisée est supérieure à 6 jusqu'à 12% en poids et la quantité d'eau utilisée est comprise entre 0,5 et 35% en poids ou bien la quantité du peroxyde utilisée est supérieure à 12 jusqu'à 15% en poids et la quantité d'eau utilisée est comprise entre 0,5 et 25% en poids ou bien la quantité du peroxyde utilisée est supérieure à 15 jusqu'à 20% en poids et la quantité d'eau utilisée est comprise entre 0,5 et 15% en poids ou bien la quantité du peroxyde utilisée est supérieure à 20 jusqu'à 25% en poids et la quantité d'eau utilisée est comprise entre 0,5 et 10% en poids, les pourcentages pondéraux ci-dessus étant basés sur le poids total du méthanol, du peroxyde organique, du formaldéhyde et de l'eau présents dans le mélange réactionnel.

2. Procédé selon la revendication 1 où le peroxyde organique est du peroxyde de dibutyle tertiaire.

3. Procédé selon la revendication 1 ou 2 où la durée de la réaction ne dépasse pas 8 heures.

4. Procédé selon la revendication 3 où le mélange réactionnel initial contient du peroxyde de dibutyle tertiaire comme peroxyde, de l'eau, 0,5 à 13% en poids de formaldéhyde et le restant étant du méthanol, la température de la réaction est comprise entre 100 et 200°C et la durée de la réaction est comprise entre 0,25 et 8 heures.

5. Procédé selon la revendication 4 où la quantité de formaldéhyde est de 2 à 12% en poids, la température de la réaction est comprise entre 125 et 175°C et la durée de la réaction est comprise entre 1/2 heure et 4 heures.

6. Procédé selon la revendication 5 où le mélange réactionnel initial contient 41 à 91,5% de

méthanol, plus de 6 jusqu'à 12% en poids de peroxyde de dibutyle tertiaire et de 0,5 à 35% en poids d'eau.

7. Procédé selon la revendication 5 où le mélange réactionnel initial contient de 48 à 85,5% en poids de méthanol, plus de 12 jusqu'à 15% en poids de peroxyde de dibutyle tertiaire et de 0,5 à 25% en poids d'eau.

8. Procédé selon la revendication 5 où le mélange réactionnel initial contient de 53 à 82,5% en poids de méthanol, plus de 15 jusqu'à 20% en poids de peroxyde de dibutyle tertiaire et de 0,5 à 15% en poids d'eau.

9. Procédé selon la revendication 5 où le mélange réactionnel initial contient de 53 à 77,5% en poids de méthanol, plus de 20 jusqu'à 25% en poids de peroxyde de dibutyle tertiaire et de 0,5 à 10% en poids d'eau.

10. Procédé selon la revendication 5 où le mélange réactionnel initial contient de 68 à 87% en poids de méthanol, plus de 6 jusqu'à 10% en poids de peroxyde de dibutyle tertiaire et de 5 à 10% en poids d'eau.

**Patentansprüche**

1. Verfahren zur Herstellung von Ethylenglycol durch Reaktion von Methanol, einem organischen Peroxid und Formaldehyd in Gegenwart von Wasser, wobei das organische Peroxid die Formel R—O—O—$R^1$ besitzt, in der R und $R^1$ jeweils (gleich oder verschieden sein können und) eine Alkyl- oder Aralkyl-Gruppe mit 3 bis 12 Kohlenstoff-Atomen darstellen, dadurch gekennzeichnet, daß die Menge des verwendeten Peroxids größer als 6 Gew.-% ist und bis zu 25 Gew.-% beträgt und die Menge des verwendeten Wassers 0,5 bis 35 Gew.-% beträgt sowie die Mengen des verwendeten Peroxids und des verwendeten Wassers voneinander in der Weise abhängen, daß entweder die Menge des verwendeten Peroxids größer als 6 Gew.-% ist und bis zu 12 Gew.-% beträgt und die Menge des verwendeten Wassers 0,5 bis 35 Gew.-% beträgt oder die Menge des verwendeten Peroxids größer als 12 Gew.-% ist und bis zu 15 Gew.% beträgt und die Menge des verwendeten Wassers 0,5 bis 25 Gew.-% beträgt oder die Menge des verwendeten Peroxids größer als 15 Gew.-% ist und bis zu 20 Gew.-% beträgt und die Menge des verwendeten Wassers 0,5 bis 15 Gew.-% beträgt oder die Menge des Verwendeten Peroxids größer als 20 Gew.-% ist und bis zu 25 Gew.-% beträgt und die Menge des verwendeten Wassers 0,5 bis 10 Gew.-% beträgt, wobei die Vorstehenden Angaben in Gew.-% sich auf das Gesamtgewicht des Methanols, organischen Peroxids, Formaldehyds und Wassers, die in der Reaktionsmischung vorhanden sind, beziehen.

2. Verfahren nach Anspruch 1, dadurch gekennzeichnet, daß das organische Peroxid Di-tert.-butylperoxid ist.

3. Verfahren nach Anspruch 1 oder 2, dadurch gekennzeichnet, daß die Reaktionszeit nicht größer ist als 8 h.

4. Verfahren nach Anspruch 3, dadurch gekennzeichnet, daß die anfängliche Reaktionsmischung Di-tert.-butylperoxid als das Peroxid, Wasser, 0,5 bis 13 Gew.-% Formaldehyd und als Rest Methanol enthält, die Reaktionstemperatur 100°C bis 200°C beträgt und die Reaktionszeit 0,25 bis 8 h beträgt.

5. Verfahren nach Anspruch 4, dadurch gekennzeichnet, daß die Formaldehyd-Menge 2 bis 12 Gew.-% beträgt, die Reaktionstemperatur 125°C bis 175°C beträgt und die Reaktionszeit 0,5 bis 4 h beträgt.

6. Verfahren nach Anspruch 5, dadurch gekennzeichnet, daß die anfängliche Reaktionsmischung 41 bis 91,5 Gew.-% Methanol, mehr als 6 bis zu 12 Gew.-% Di-tert.-butylperoxid und 0,5 bis 35 Gew.-% Wasser enthält.

7. Verfahren nach Anspruch 5, dadurch gekennzeichnet, daß die anfängliche Reaktionsmischung 48 bis 85,5 Gew.-% Methanol, mehr als 12 bis zu 15 Gew.-% Di-tert.-butylperoxid und 0,5 bis 25 Gew.-% Wasser enthält.

8. Verfahren nach Anspruch 5, dadurch gekennzeichnet, daß die anfängliche Reaktionsmischung 53 bis 82,5 Gew.-% Methanol, mehr als 15 bis zu 20 Gew.-% Di-tert.-butylperoxid und 0,5 bis 15 Gew.-% Wasser enthält.

9. Verfahren nach Anspruch 5, dadurch gekennzeichnet, daß die anfängliche Reaktionsmischung 53 bis 77,5 Gew.-% Methanol, mehr als 20 bis zu 25 Gew.-% Di-tert.-butylperoxid und 0,5 bis 10 Gew.-% Wasser enthält.

10. Verfahren nach Anspruch 5, dadurch gekennzeichnet, daß die anfängliche Reaktionsmischung 68 bis 87 Gew.-% Methanol, mehr als 6 bis zu 10 Gew.-% Di-tert.-butylperoxid und 5 bis 10 Gew.-% Wasser enthält.

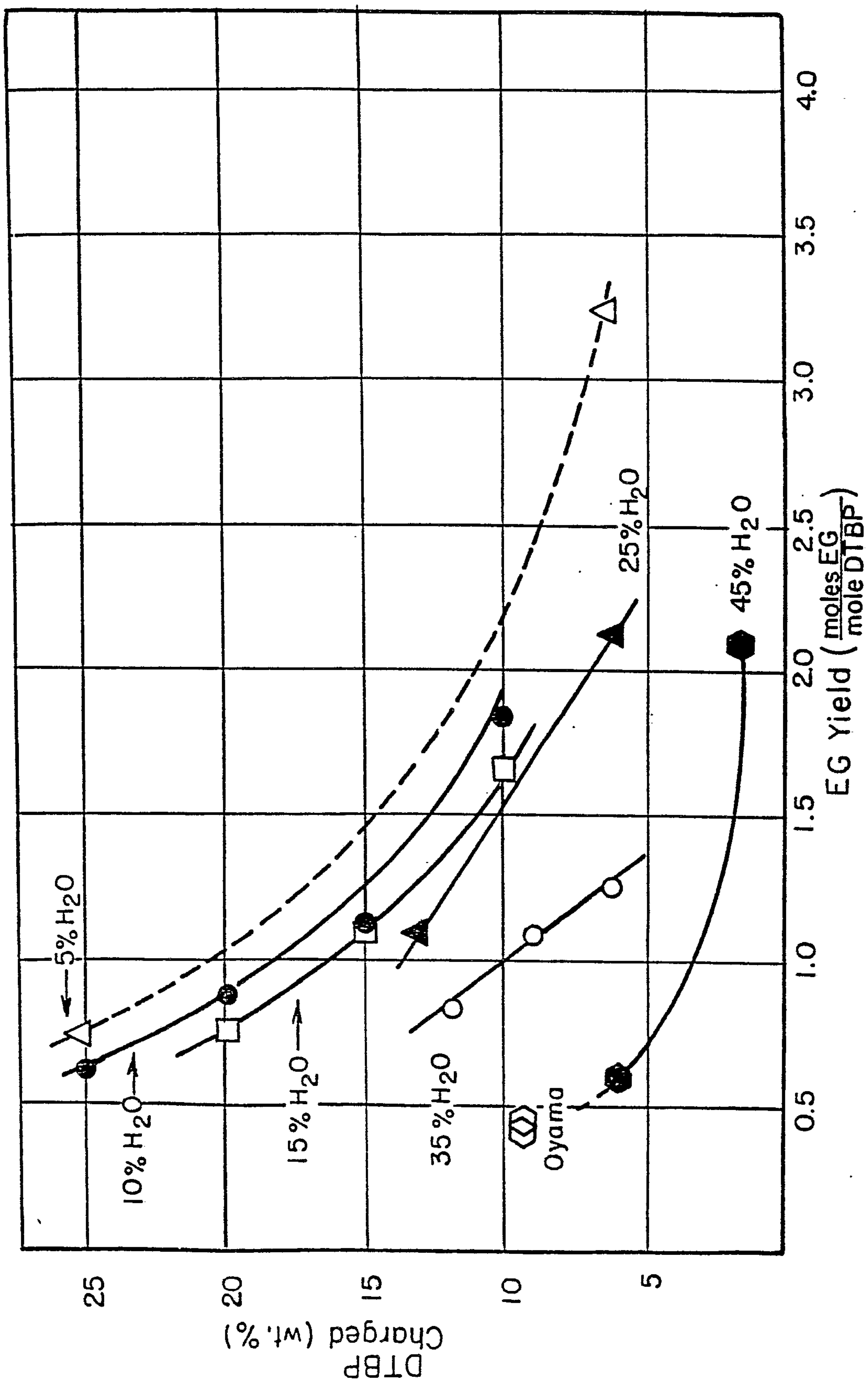

DTBP Charged (wt. %)
EG Yield (moles EG / mole DTBP)
5% $H_2O$
10% $H_2O$
15% $H_2O$
25% $H_2O$
35% $H_2O$
45% $H_2O$
Oyama
0.5
1.0
1.5
2.0
2.5
3.0
3.5
4.0
5
10
15
20
25